# EUROPÄISCHE PATENTSCHRIFT

(11) **EP 2 131 847 B1**
(45) Veröffentlichungstag und Bekanntmachung des Hinweises auf die Patenterteilung: **29.12.2010**
(21) Anmeldenummer: 08716068.5
(22) Anmeldetag: 27.02.2008
(51) Int. Cl.: A61K 31/57, A61K 31/565, A61P 17/00

(54) **ARZNEIMITTEL UMFASSEND WENIGSTENS EIN GESTAGEN**
DRUG COMPRISING AT LEAST ONE GESTAGEN
MÉDICAMENT CONTENANT AU MOINS UN GESTAGÈNE

(30) Priorität: 07.03.2007 DE 102007011486
(43) Veröffentlichungstag der Anmeldung: 16.12.2009
(73) Patentinhaber: Grünenthal GmbH, 52078 Aachen (DE)
(72) Erfinder: SCHRAMM, Georg, 52224 Stolberg (DE)
(74) Vertreter: Kutzenberger, Helga
(86) Internationale Anmeldenummer: PCT/EP2008/001532
(87) Internationale Veröffentlichungsnummer: WO 2008/107099

(56) Entgegenhaltungen:
- WO-A-00/38691
- WO-A-02/22110
- WO-A-2006/013464
- WO-A-2007/002862
- WO-A-2007/085420
- WO-A-2007/098828
- DE-A1- 1 568 062
- DE-A1- 4 339 934
- DE-A1- 19 739 916
- US-A- 4 171 358
- BUNDESVERBAND DER PHARMAZEUTISCHEN INDUSTRIE E V UND WEITERE ED - ROTE LISTE SERVICE GMBH (ED): "Rote Liste 2002, passage" ROTE LISTE 2002. ARZNEIMITTELVERZEICHNIS FUER DEUTSCHLAND (EINSCHLIESLICH EU - ZULASSUNGEN UND BESTIMMTER MEDIZINPRODUKTE); [ROTE LISTE], AULENDORF : EDITIO CANTOR, DE, 1. Januar 2002 (2002-01-01), Seiten 76-131, XP002432757 ISBN: 978-3-87193-252-6
- ANONYM: "Chlormadinon 2 mg JENAPHARM Tbl" GELBE LISTE PHARMINDEX, XX, XX, 1. Januar 2006 (2006-01-01), XP002435839
- HONMA S ET AL: "IDENTIFICATION AND ANTI-ANDROGENIC ACTIVITY OF THE METABOLITES OF 17ALPHA-ACETOXY-6-CHLOROPREGNA-4,6-DIENE-3 ,20-DIONE (CHLORMADINONE ACETATE) IN THE RAT, RABBIT, DOG AN DMAN" CHEMICAL AND PHARMACEUTICAL BULLETIN, PHARMACEUTICAL SOCIETY OF JAPAN, TOKYO, Bd. 25, Nr. 8, 25. August 1977 (1977-08-25), Seiten 2019-2031, XP009083454 ISSN: 0009-2363

## Beschreibung

Die vorliegende Erfindung betrifft die Verwendung von wenigstens einem Gestagen ausgewählt aus Chlormadinonacetat, 3α-Hydroxy-6-chlor-17α-acetoxy-4,6-pregnadien-20-on (3α-Hydroxy-chlormadinonacetat) und 3β-Hydroxy-6-chlor-17α-acetoxy-4,6-pregnadien-20-on (3β-Hydroxy-chlormadinonacetat) und ggf. wenigstens einem Östrogen ausgewählt aus Ethinylestradiol, Estron, Estriol und Estradiol zur Herstellung eines Arzneimittels zur Behandlung und/oder zur Prophylaxe von Melasma und gegebenenfalls zur gleichzeitigen hormonalen Kontrazeption oder ggf. zum gleichzeitigen Hormonersatz bei Frauen.

Eine sehr häufige Nebenwirkung, die bei der Einnahme von Hormonpräparaten, insbesondere bei der Einnahme von Kontrazeptiva, beobachtet werden kann, ist eine Hyperpigmentierung der Gesichtshaut in Form von bräunlich-grauen Pigmentflecken, häufig in Form von Girlanden, die auch unter dem Begriff Melasma (früher: Chloasma) bekannt ist. Für diese Hyperpigmentierung wird ein erhöhter Östrogen-Spiegel verantwortlich gemacht.

Eine derartige Hyperpigmentierung der Gesichtshaut stellt ein großes Problem dar, da die Hautflecken als äußerst störend empfunden werden und die Lebensqualität der Betroffenen dadurch zum Teil stark beeinträchtigt wird.

Erschwerend kommt hinzu, dass eine Behandlung dieser Pigmentstörung langwierig und oft nur wenig zufriedenstellend ist. Bislang sind keine wirksamen Therapeutika zur Behandlung von Melasma bekannt.

Es besteht daher ein Bedarf, ein Arzneimittel zur Verfügung zu stellen, das bei Frauen zur Behandlung und/oder zur Prophylaxe von Melasma und ggf. gleichzeitiger, hormonaler Kontrazeption oder ggf. zum bei gleichzeitigen Hormonersatz vor allem in der Peri- oder Postmenopause geeignet ist.

Diese Aufgabe wird durch die Verwendung von wenigstens einem Gestagen ausgewählt aus Chlormadinonacetat, 3α-Hydroxy-6-chlor-17α-acetoxy-4,6-pregnadien-20-on (3α-Hydroxy-chlormadinonacetat) und 3β-Hydroxy-6-chlor-17α-acetoxy-4,6-pregnadien-20-on (3β-Hydroxy-chlormadinonacetat) und ggf. von wenigstens einem Östrogen ausgewählt aus Ethinylestradiol, Estron, Estriol und Estradiol zur Herstellung eines Arzneimittels zur Behandlung und/oder zur Prophylaxe von Melasma und gegebenenfalls zur gleichzeitigen hormonalen Kontrazeption oder ggf. zum gleichzeitigen Hormonersatz bei Frauen gelöst.

Das Arzneimittel wird vorzugsweise in Form von Tages-Einheiten bereitgestellt, die vorzugsweise in Form von Tabletten formuliert sind. Vorzugsweise enthalten die hormonhaltigen Tageseinheiten als Gestagenkomponente jeweils nur wenigstens eines der genannten Gestagene.

Zur Behandlung bzw. Prophylaxe von Melasma enthalten die Tages-Einheiten vorzugsweise jeweils als hormonalen Wirkstoff wenigstens eines der zuvor genannten Gestagene in einer Menge von vorzugsweise 10 mg, besonders bevorzugt 2 bis 5 mg und ganz besonders bevorzugt 2,5 bis 4 mg.

Ferner können die vorstehend genannten Gestagene in jedem beliebigen Mischungsverhältnis eingesetzt werden.

In einer bevorzugten Ausführungsform der vorliegenden Erfindung ist die erfindungsgemäß zum Einsatz kommende Gestagenkomponente vorzugsweise als einzige Hormonkomponente des erfindungsgemäßen Arzneimittels eine der folgenden Komponenten a) bis e)
a) Chlormadinonacetat,
b) 3α-Hydroxy-chlormadinonacetat,
c) 3β-Hydroxy-chlormadinonacetat,
d) eine Mischung von b) und c) in einem beliebigen Mischungsverhältnis,
e) eine Mischung von a), b) und/oder c) in Mischungsverhältnis von 10 bis 90 Gew.% Chlormadinonacetat und 90 bis 10 Gew.%, bezogen auf die Gesamtmischung der übrigen Gestagene.

Sofern gleichzeitig auch ein ausreichender kontrazeptiver Schutz bei Frauen erzielt werden soll und wenigstens eines der zuvor genannten Gestagene als einzige Gestagenkomponente oder einzige Hormonkomponente verwendet wird, werden vorzugsweise zur Herstellung einer Tages-Einheit 1, 2, 3, 4 oder 5 mg Chlormadinonacetat, 3α-Hydroxy-chlormadinonacetat und/oder 3β-Hydroxy-chlormadinonacetat und gegebenenfalls übliche Hilfsstoffe eingesetzt.

Wird neben der Behandlung von Melasma auch gleichzeitig eine Hormonersatztherapie durchgeführt, enthalten die hormonhaltigen Tageseinheiten jeweils wenigstens eines der zuvor genannten Gestagene als einzige Hormonkomponente, vorzugsweise 1, 2, 3, 4 oder 5 mg Chlormadinonacetat, 3α-Hydroxy-chlormadinonacetat und/oder 3β-Hydroxy-chlormadinonacetat und gegebenenfalls übliche Hilfsstoffe.

Besonders bevorzugt wird zur Behandlung und/oder Prophylaxe von Melasma und ggf. einer gleichzeitigen hormonalen Kontrazeption oder ggf. eines gleichzeitigen Hormonersatz Chlormadinonacetat als vorzugsweise einziger, hormonaler Wirkstoff eingesetzt.

Die Anzahl der Tages-Einheiten eines Arzneimittels, das nur wenigstens eines der zuvor genannten Gestagene als einzige Hormonkomponente enthält, kann wenigstens einem natürlichen, monatlichen, weiblichen Menstruations-Zyklus entsprechen. Dazu wird das Arzneimittel vorzugsweise in Form von mindestens 28 wenigstens eines der genannten Gestagene als hormonalen Wirkstoff enthaltenden Tages-Einheiten zur ununterbrochenen oralen, täglichen Verabreichung bereitgestellt. Es ist aber auch möglich, dass die Gesamtzahl der diesen hormonalen Wirkstoff enthaltenden Tages-Einheiten größer ist als es einem natürlichen, weiblichen Monatszyklus entspricht, sodass eine vorzugsweise ununterbrochene Einnahme des Arzneimittels von bis zu zwei Jahren, vorzugsweise bis zu 1 Jahr zur Behandlung von Melasma und ggf. zur gleichzeitigen hormonalen Kontrazeption oder ggf. zum gleichzeitigen Hormonersatz möglich ist.

In einer bevorzugten Ausführungsform der vorliegenden Erfindung wird das Arzneimittel, welches als vorzugsweise einzige Hormonkomponente wenigstens eines der zuvor genannten Gestagene beinhaltet, in Form von 28, 56, 84, 112, 140, 168, 196, 224, 252, 280, 308, 336 oder 364 Tages-Einheiten zur ununterbrochenen oralen, täglichen Verabreichung bereitgestellt.

Das erfindungsgemäße Arzneimittel, welches als vorzugsweise einzige Hormonkomponente wenigstens eines der zuvor genannten Gestagene beinhaltet, kann auch ein Bestandteil eines Kits sein, wobei ein derartiger Kit entsprechend dem weiblichen Menstruationszyklus von 28 Tagen vorzugsweise mindestens 28 Tages-Einheiten umfasst, die vorzugsweise zusammen in einem Blister und vorzugsweise unter Kennzeichnung der jeweils einzunehmenden Tages-Einheit verpackt sind. Es können jedoch auch 1, 2, 3, 4, 5, 6, 7, 8, 9, 10, 11, 12 , 13,14 oder 15 Blister zu jeweils 28 Tages-Einheiten, welche als Hormonkomponente jeweils wenigstens eines der zuvor genannten Gestagene vorzugsweise als einzige Hormonkomponente beinhalten, in einem Kit enthalten sein. Gegebenenfalls umfasst der Kit auch noch ein Kalender bzw. ein Kalenderbuch.

Vorzugsweise enthalten die gestagenhaltigen Tages-Einheiten jeweils dieselbe Menge wenigstens eines der genannten Gestagene.

In einer weiteren bevorzugten Ausführungsform enthält das Arzneimittel zur Behandlung und/oder Prophylaxe von Melasma und ggf. zur gleichzeitigen hormonalen Kontrazeption oder ggf. zum gleichzeitigen Hormonersatz eine hormonale Wirkstoffkombination aus wenigstens einem Gestagen ausgewählt aus Chlormadinonacetat, 3α-Hydroxy-chlormadinonacetat und 3β-Hydroxy-chlormadinonacetat vorzugsweise als einzige Gestagenkomponente und wenigstens einem Östrogen ausgewählt aus Ethinylestradiol, Estron, Estriol und Estradiol vorzugsweise als einzige Östrogenkomponente.

Als Wirkstoffkombination zur Herstellung einer Tages-Einheit werden vorzugsweise jeweils eine Hormonkombination aus vorzugsweise 0,001 bis 50 µg, besonders bevorzugt 5 bis 50 µg, ganz besonders bevorzugt 5 bis 30 µg Ethinylestradiol bzw. vorzugsweise 0,01 bis 60 mg, besonders bevorzugt 0,5 bis 50 mg, ganz besonders bevorzugt 1 bis 50 mg Estron bzw. vorzugsweise 0,01 bis 60 mg, besonders bevorzugt 0,5 bis 50 mg, ganz besonders bevorzugt 1 bis 50 mg Estriol, oder vorzugsweise 0,1 bis 5 mg, besonders bevorzugt 0,5 bis 3 mg, ganz besonders bevorzugt 1 bis 2 mg Estradiol als vorzugsweise einzige Östrogenkomponente und 1 bis 10 mg, besonders bevorzugt 2 bis 5 mg, ganz besonders bevorzugt 1 bis 5 mg Chlormadinonacetat, 3α-Hydroxy-chlormadinonacetat und/oder 3β-Hydroxy-chlormadinonacetat als vorzugsweise einzige Gestagenkomponente und gegebenenfalls übliche Hilfsstoffe eingesetzt.

In einer besonders bevorzugten Ausführungsform der vorliegenden Erfindung werden als Wirkstoffkombination zur Herstellung einer Tages-Einheit eine Hormonkombination bestehend aus vorzugsweise 0,001 bis 50 µg, besonders bevorzugt 5 bis 50 µg, ganz besonders bevorzugt 5 bis 30 µg Ethinylestradiol oder vorzugsweise 0,1 bis 5 mg, besonders bevorzugt 0,5 bis 3 mg, ganz besonders bevorzugt 1 bis 2 mg Estradiol und 1 bis 10 mg, besonders bevorzugt 2 bis 5 mg, ganz besonders bevorzugt 1 bis 5 mg Chlormadinonacetat, 3α-Hydroxy-chlormadinonacetat und/oder 3β-Hydroxy-chlormadinonacetat und gegebenenfalls übliche Hilfsstoffe eingesetzt.

Sofern neben einer Behandlung und/oder Prophylaxe von Melasma auch gleichzeitig ein ausreichender, kontrazeptiver Schutz oder Hormonersatz bei Frauen erzielt werden soll, werden vorzugsweise zur Herstellung einer Tages-Einheit eine Hormonkombination bestehend aus jeweils 15 µg, 20 µg oder 30 µg Ethinylestradiol oder 1 oder 2 mg Estradiol und jeweils 1, 2, 3, 4 oder 5 mg Chlormadinonacetat, 3α-Hydroxy-chlormadinonacetat und/oder 3β-Hydroxy-chlormadinonacetat und gegebenenfalls übliche Hilfsstoffe eingesetzt.

In einer besonders bevorzugten Ausführungsform der vorliegenden Erfindung wird zur Behandlung und/oder zur Prophylaxe von Melasma und zur Erzielung eines gleichzeitigen Kontrazeption pro Tageseinheit eine hormonale Wirkstoffkombination bestehend aus jeweils 20 µg Ethinylestradiol und ≥ 2 mg Chlormadinonacetat, 3α-Hydroxy-chlormadinonacetat und/oder 3β-Hydroxy-chlormadinonacetat verwendet.

Soll neben der erfindungsgemäßen Behandlung von Melasma auch gleichzeitig eine Hormonersatztherapie durchgeführt werden, so enthält eine Tages-Einheit vorzugsweise eine hormonale Wirkstoffkombination bestehend aus Estradiol und Chlormadinonacetat, 3α-Hydroxy-chlormadinonacetat und/oder 3β-Hydroxy-chlormadinonacetat. Bevorzugt enthält ein derartiges Arzneimittel 0,5 bis 3 mg, besonders bevorzugt 1 bis 2 mg Estradiol und 1 bis 10 mg, besonders bevorzugt 1 bis 5 mg Chlormadinonacetat, 3α-Hydroxy-chlormadinonacetat und/oder 3β-Hydroxy-chlormadinonacetat und gegebenenfalls übliche Hilfsstoffe.

Vorzugsweise enthalten die hormonhaltigen Tages-Einheiten dieselbe Hormonkombination in derselben Menge mit demselben Anteil der Gestagen- bzw. Östrogenkomponente.

Enthält das erfindungsgemäß zum Einsatz kommende Arzneimittel eine wie vorstehend beschriebene hormonale Wirkstoffkombination, so wird es in Form von mindestens 21 eine der vorstehend aufgeführte Wirkstoffkombinationen als Hormonkombination enthaltenden Tages-Einheiten zur ununterbrochenen Verabreichung, gegebenenfalls gefolgt von einer 7 bis 3-tägigen Einnahmepause oder von 7 bis 3 hormonfreien Tages-Einheiten zur ununterbrochenen Verabreichung bereitgestellt.

Zur Behandlung bzw. zur Prophylaxe von Melasma und gegebenenfalls zur gleichzeitigen hormonalen Kontrazeption oder ggf. zum gleichzeitigen Hormonersatz kann das Arzneimittel in Form von jeweils eine der vorstehend aufgeführten Hormonkombinationen als Wirkstoffkombination enthaltenden Tages-Einheiten auch für eine ununterbrochene Verabreichung über mehrere Jahre, vorzugsweise bis zu 2 Jahren, besonders bevorzugt bis zu 1 Jahr, gegebenenfalls in Kombination mit 7 bis 3 hormonfreien Tages-Einheiten zur ununterbrochenen, anschließenden Verabreichung oder gefolgt von einer 7 bis 3-tägigen Einnahmepause bereitgestellt werden.

Das erfindungsgemäß zubereitete Arzneimittel kann aber auch für die erfindungsgemäße Therapierung in einer Darreichungsform mit weniger als 365 einer der vorstehend aufgeführten hormonalen Wirkstoffkombinationen enthaltenden Tages-Einheiten, wie z. B. mit 77 bis 193 bzw. 42 bis 52 einer der vorstehend aufgeführten hormonalen Wirkstoffkombinationen enthaltenden Tages-Einheiten zur ununterbrochenen oralen Verabreichung, gefolgt von einer Einnahmepause über 7 bis 3 Tage oder von 7 bis 3 hormonfreien Tages-Einheiten zur ununterbrochenen Verabreichung bereitgestellt werden.

Bei einer vorgesehenen Einnahme von bis zu 2 Jahren kann die vorstehend genannte Darreichungsform, welche die eine der vorstehend aufgeführten Wirkstoffkombinationen enthaltenden Tages-Einheiten und ggf. die vorstehend aufgeführten hormonfreien Tageseinheiten aufweist, in einer entsprechenden Verpackung zur Verfügung gestellt werden.

In einer weiteren bevorzugten Ausführungsform der vorliegenden Erfindung kann das erfindungsgemäß zum Einsatz kommende Arzneimittel in Form von 21 bis 25 einer der vorstehend aufgeführten Wirkstoffkombinationen als Hormonkombination enthaltenden Tages-Einheiten zur ununterbrochenen Verabreichung und gefolgt von 7 bis 3 hormonfreien Tages-Einheiten zur anschließenden ununterbrochenen Verabreichung oder gefolgt von einer Einnahmepause von 3 bis 7 Tagen bereitgestellt werden.

Dementsprechend kann das erfindungsgemäß verwendete Arzneimittel mit der vorstehend aufgeführten Anzahl von hormonhaltigen und hormonfreien Tages-Einheiten auch in einer Verpackung vorliegen. Dabei sind die eine der vorstehend aufgeführten Wirkstoffkombinationen als Hormonkombination enthaltenden Tages-Einheiten und die hormonfreien Tages-Einheiten vorzugsweise in Blistern verpackt. Sofern die Anzahl der in einem Blister vorliegenden Tages-Einheiten, vorzugsweise unter Kennzeichnung der jeweils einzunehmenden Tages-Einheit, vorzugsweise entsprechend einem weiblichen Zyklus von 28 Tagen, können auch mehrere Blister zu einem Kit zusammengefasst sein, beispielsweise zu 1, 2, 3, 4, 5, 6, 7, 8, 9, 10, 11, 12, 13, 14 oder 15 Blistern, wobei jedes Blister jeweils eine wie vorstehend ausgeführte Kombination aus hormonhaltigen und hormonfreien Tages-Einheiten für eine ununterbrochene Verabreichung beinhaltet.

In einer besonders bevorzugten Ausführungsform der vorliegenden Erfindung weist das Arzneimittel in jeder, eine der genannten Wirkstoffkombinationen als Hormonkombination enthaltenden Tages-Einheit mengenmäßig dieselbe Hormonkombination bestehend aus wenigstens einem Östrogen vorzugsweise ausgewählt aus Ethinylestradiol und Estradiol und wenigstens einem Gestagen ausgewählt aus Chlormadinonacetat, 3α-Hydroxy-chlormadinonacetat und 3β-Hydroxy-chlormadinonacetat auf, wobei auch die Zusammensetzung der jeweiligen Hormonkombinationen mengenmäßig nicht variiert.

Die vorzugsweise als Tabletten vorliegenden Tages-Einheiten des erfindungsgemäßen Arzneimittels können nach üblichen, dem Fachmann bekannten Formulierungsverfahren hergestellt werden.

### Beispiele

### a) Herstellung des Arzneimittels

### Beispiel 1:

### Zusammensetzung

| | Pro Tablette | Pro Charge |
|---|---|---|
| Ethinylestradiol | 0,020 mg | 0,0020 kg |
| Chlormadinonacetat | 2,000 mg | 0,2000 kg |
| Povidon K30 | 3,000 mg | 0,3000 kg |
| Lactose | 32,980 mg | 3,2980 kg |
| Maisstärke | 12,000 mg | 1,2000 kg |
| Magnesiumstearat | 0,500 mg | 0,0500 kg |
| Hochdisperses Siliciumdioxid | 0,500 mg | 0,0500 kg |

Ethinylestradiol (EE) und Povidone K 30 (PVP) wurden in 600 ml Ethanol gelöst. Chlormadinonacetat (Partikelgröße 90% <50µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepresst.

Die Tabletten wurden mit einem Lack auf Basis Methylhydroxypropylcellulose mit folgender Zusammensetzung überzogen (Überzugsmasse 2 mg pro Tablette)

| | |
|---|---|
| Methylhydroxypropylcellulose 6 mPa·s, | 0,1351 kg |
| Polyethylenglykol 6000 | 0,0395 kg |
| Propylenglykol | 0,0054 kg |
| Gereinigtes Wasser | 1,6200 kg |

Jeweils 24 Tabletten wurden als die hormonale Wirkstoffkombination enthaltenden Tages-Einheiten und jeweils entsprechend zusammengesetzte 4 hormonfreie Tabletten in einem Blister verpackt.

### Beispiel 2:

Analog zu Beispiel 1 wurden Tabletten mit folgender Zusammensetzung hergestellt:

| | Pro Tablette | Pro Charge |
|---|---|---|
| Ethinylestradiol | 0,030 mg | 0,0030 kg |
| Chlormadinonacetat | 2,000 mg | 0,2000 kg |
| Povidon K30 | 3,000 mg | 0,3000 kg |
| Lactose | 32,970 mg | 3,2970 kg |
| Maisstärke | 12,000 mg | 1,2000 kg |
| Magnesiumstearat | 0,500 mg | 0,0500 kg |
| Hochdisperses Siliciumdioxid | 0,500 mg | 0,0500 kg |

Jeweils 21 Tabletten wurden als die hormonale Wirkstoffkombination enthaltenden Tages-Einheiten in einem Blister verpackt.

### b) In vitro-Daten

In einer Serie von Experimenten wurde der Einfluss von Chlormadinonacetat (CMA) auf die Proliferation bzw. die Pigmentbildung von humanen Melanozyten überprüft.

Hierzu wurden humane Melanozyten jeweils im entsprechenden Kulturmedium kultiviert, das jeweils unterschiedliche Mengen an Ethinylestradiol (EE), Chlormadinonacetat (CMA) oder deren Kombination enthielt. Die einzelnen Zusammensetzungen sind nachfolgend in Tabelle 1 zusammengefasst.

**Tabelle 1**

| **Ansatz #** | **CMA** | **EE** |
|---|---|---|
| 1 | - | + |
| 2 | + | - |
| 3 | + | + |

Nach 9 Tagen wurden die humanen Melanozyten *in vitro* analysiert. Zellwachstum und Zelltod wurden über Zellzählung mit Hilfe der Typanblaufärbung sowie über kommerzielle Zellproliferations- und Apoptose-Tests bestimmt. Die Pigmentbildung wurde sowohl durch Messung der Melaninkonzentration der Zellen als auch durch Messung der Tyrosinase, dem Schlüsselenzym der Pigmentbildung, bestimmt.

Es konnte gezeigt werden, dass bei Anwesenheit von 1 nM Ethinylestradiol (vgl. Tabelle 1, Ansatz #1) als einziges Hormon im Kulturmedium die Tyrosinase-Aktivität bzw. die Proliferation von humanen Melanozyten erhöht ist.

Es konnte gezeigt, werden, dass bei einer Anwesenheit von Chlormadinonacetat (vgl. Tabelle 1, Ansatz # 2) (100 nM) im Kulturmedium, die Proliferationsrate von humanen Melanozyten um 50-70% verringert wird.

Bei humanen Melanozytenkulturen, die in Anwesenheit einer Hormonkombination (vgl. Tabelle 1, Ansatz #3) aus Ethinylestradiol und Chlormadinonacetat kultiviert wurden, konnte eine deutliche Verringerung der Proliferationsrate der Melanozyten, im Vergleich zu Melanozytenkulturen, die ausschließlich in Anwesenheit von Ethinylestradiol kultiviert wurden, beobachtet werden.

### c) in vivo-Studie

In einer Studie mit 20.897 Frauen, die eine hormonale Wirkstoffkombination aus 30 µg Ethinylestradiol und 2 mg Chlormadinonacetat pro Tageseinheit für die Dauer von 4 Menstruations-Zyklen einnahmen, wurde die erfindungsgemäße Wirkung des Arzneimittels gemäß Beispiel 2 geprüft.

Bei nur 4 von 20.897 Frauen, die an der Studie teilnahmen, trat Melasma auf. Dies sind 0,019% der teilnehmenden Frauen.

Ferner konnte gezeigt werden, dass bei teilnehmenden Frauen, die durch eine vorherige Einnahme eines eine von den erfindungsgemäßen Hormonkombinationen unterschiedlichen Hormonkombination enthaltenen hormonalen Wirkstoffpräparates bereits Melasma hatten, diese durch Einnahme des Arzneimittels gemäß Beispiel 2 geheilt werden konnten oder die diesen eine deutliche Verbesserung der Hyperpigmentierung der Gesichtshaut erzielt werden konnte.

## Patentansprüche

1. Verwendung von wenigstens einem Gestagen ausgewählt aus Chlormadinonacetat, 3α-Hydroxy-6-chlor-17α-acetoxy-4,6-pregnadien-20-on (3α-Hydroxy-chlormadinonacetat) und 3β-Hydroxy-6-chlor-17α-acetoxy-4,6-pregnadien-20-on (3β-Hydroxy-chlormadinonacetat) als Gestagenkomponente und ggf. wenigstens einem Östrogen ausgewählt aus Ethinylestradiol, Estron, Estriol und Estradiol als Östrogenkomponente zur Herstellung eines Arzneimittels zur Behandlung und/oder zur Prophylaxe von Melasma bei Frauen.

2. Verwendung gemäß Anspruch 1, **dadurch gekennzeichnet, dass** das Arzneimittel in Form von Tages-Einheiten bereitgestellt wird.

3. Verwendung gemäß Anspruch 1 oder 2, **dadurch gekennzeichnet, dass** zur Herstellung einer Tages-Einheit als einziger hormonaler Wirkstoff eine Gestagenkomponente ausgewählt aus Chlormadinonacetat, 3α-Hydroxy-chlormadinonacetat und 3β-Hydroxy-chlormadinonacetat in einer Menge von 1 bis 10 mg, vorzugsweise
2 bis 5 mg, besonders bevorzugt 1, 2, 3, 4 oder 5 mg und gegebenenfalls übliche Hilfsstoffe eingesetzt werden.

4. Verwendung gemäß Anspruch 3, **dadurch gekennzeichnet, dass** das Arzneimittel in Form von 28, 56, 84, 112, 140, 168, 196, 224, 252, 280, 308, 336 oder 364 Tages-Einheiten zur ununterbrochenen oralen, täglichen Verabreichung bereitgestellt wird.

5. Verwendung gemäß Anspruch 1, **dadurch gekennzeichnet, dass** als Wirkstoffkombination eine Hormonkombination bestehend aus wenigstens einem Gestagen ausgewählt aus Chlormadinonacetat, 3α-Hydroxy-6-chlor-17α-acetoxy-4,6-pregnadien-20-on (3α-Hydroxy-chlormadinonacetat) und 3β-Hydroxy-6-chlor-17α-acetoxy-4,6-pregnadien-20-on (3β-Hydroxy-chlormadinonacetat) als Gestagenkomponente und aus wenigstens einem Östrogen ausgewählt aus Ethinylestradiol, Estron, Estriol und Estradiol als Östrogenkomponente eingesetzt wird.

6. Verwendung gemäß Anspruch 5, **dadurch gekennzeichnet, dass** zur Herstellung einer Tages-Einheit eine Hormonkombination bestehend aus 5 bis 50 µg Ethinylestradiol, 0,5 bis 3 mg Estradiol, 0,5 bis 50 mg Estron oder 0,5 bis 50 mg Estriol und 1 bis 5 mg der Gestagenkomponente und gegebenenfalls übliche Hilfsstoffe eingesetzt werden.

7. Verwendung gemäß Anspruch 5 oder 6, **dadurch gekennzeichnet, dass** zur Herstellung einer Tages-Einheit eine Hormonkombination bestehend aus 5 bis 30 µg Ethinylestradiol oder 1 bis 2 mg Estradiol und 1 bis 5 mg der Gestagenkomponente und gegebenenfalls übliche Hilfsstoffe eingesetzt werden.

8. Verwendung gemäß einem der Ansprüche 5 bis 7, **dadurch gekennzeichnet, dass** zur Herstellung einer Tages-Einheit eine Hormonkombination bestehend aus jeweils 15 µg, 20 µg oder 30 µg Ethinylestradiol oder jeweils 1 oder 2 mg Estradiol und jeweils 1, 2, 3, 4 oder 5 mg der Gestagenkomponente und gegebenenfalls übliche Hilfsstoffe eingesetzt werden.

9. Verwendung gemäß einem der Ansprüche 5 bis 8, **dadurch gekennzeichnet, dass** zur Herstellung Tageseinheit eine Hormonkombination aus jeweils 20 µg Ethinylestradiol und 2, 3, 4 oder 5 mg der Gestagenkomponente eingesetzt werden.

10. Verwendung gemäß einem der Ansprüche 5 bis 9, **dadurch gekennzeichnet, dass** das Arzneimittel in Form von mindestens 21, eine der Hormonkombinationen als Wirkstoffkombination enthaltenden Tages-Einheiten zur ununterbrochenen Verabreichung gegebenenfalls gefolgt von einer 7 bis 3-tägigen Einnahmepause oder von 7 bis 3 hormonfreien Tages-Einheiten,zur ununterbrochenen Verabreichung bereitgestellt wird.

11. Verwendung gemäß Anspruch 10, **dadurch gekennzeichnet, dass** das Arzneimittel in Form von eine der Hormonkombinationen als Wirkstoffkombination enthaltenden Tages-Einheiten für eine ununterbrochene Verabreichung über mehrere Jahre, vorzugsweise bis zu 2 Jahren, besonders bevorzugt bis zu 1 Jahr, gefolgt von 7 bis 3 hormonfreien Tages-Einheiten zur ununterbrochenen Verabreichung oder gefolgt von einer Einnahmepause von 3 bis 7 Tagen bereitgestellt wird.

12. Verwendung gemäß Anspruch 11, **dadurch gekennzeichnet, dass** das Arzneimittel in Form von 77 bis 193 oder von 42 bis 52 oder von 21 bis 25, eine der Hormonkombinationen als Wirkstoffkombination enthaltenden Tages-Einheiten zur ununterbrochenen Verabreichung gefolgt von 7 bis 3 hormonfreien Tages-Einheiten zur ununterbrochenen Verabreichung oder gefolgt von einer 3 bis 7-tägigen Einnahmepause bereitgestellt wird.

13. Verwendung gemäß einem der Ansprüche 10 bis 12, **dadurch gekennzeichnet, dass** das Arzneimittel in jeder, eine der Hormonkombinationen als Wirkstoffkombination enthaltenden Tages-Einheit mengenmäßig dieselbe Kombination aus Östrogenkomponente und Gestagenkomponente aufweist.

14. Verwendung gemäß einem der Ansprüche 1 bis 13 zur Hemmung der Proliferation von menschlichen Melanozyten.

15. Verwendung gemäß einem der Ansprüche 1 bis 13 zur Verringerung des Melaningehaltes in menschlichen Melanozyten.

## Claims

1. Use of at least one gestagen selected from the group consisting of chlormadinone acetate, 3α-hydroxy-6-chloro-17α-acetoxy-4,6-pregnadien-20-one (3α-hydroxy-chlormadinone acetate) and 3β-hydroxy-6-chloro-17α-acetoxy-4,6-pregnadien-20-one (3β-hydroxy-chlormadinone acetate) as gestagen component and optionally at least one oestrogen selected from the group consisting of ethinyl oestradiol, oestrone, oestriol and oestradiol as oestrogen component to produce a medicament for the treatment and/or prevention of melasma in women.

2. Use according to claim 1, **characterised in that** the medicament is provided in the form of daily units.

3. Use according to claim 1 or claim 2, **characterised in that** a daily unit is produced by using as the sole hormonal active ingredient a gestagen component selected from the group consisting of chlormadinone acetate, 3α-hydroxy-chlormadinone acetate and 3β-hydroxy-chlormadinone acetate in a quantity of from 1 to 10 mg,
preferably 2 to 5 mg, particularly preferably of 1, 2, 3, 4 or 5 mg and optionally conventional auxiliary materials.

4. Use according to claim 3, **characterised in that** the medicament is provided in the form of 28, 56, 84, 112, 140, 168, 196, 224, 252, 280, 308, 336 or 364 daily units for uninterrupted daily oral administration.

5. Use according to claim 1, **characterised in that** the active ingredient combination used comprises a hormone combination consisting of at least one gestagen selected from the group consisting of chlormadinone acetate, 3α-hydroxy-6-chloro-17α-acetoxy-4,6-pregnadien-20-one (3α-hydroxy-chlormadinone acetate) and 3β-hydroxy-6-chloro-17α-acetoxy-4,6-pregnadien-20-one (3β-hydroxy-chlormadinone acetate) as the gestagen component and at least one oestrogen selected from the group consisting of ethinyl oestradiol, oestrone, oestriol and oestradiol as the oestrogen component.

6. Use according to claim 5, **characterised in that** a daily unit is produced by using a hormone combination consisting of 5 to 50 µg of ethinyl oestradiol, 0.5 to 3 mg of oestradiol, 0.5 to 50 mg of oestrone or 0.5 to 50 mg of oestriol and 1 to 5 mg of the gestagen component and optionally conventional auxiliary substances.

7. Use according to claim 5 or claim 6, **characterised in that** a daily unit is produced by using a hormone combination consisting of 5 to 30 µg of ethinyl oestradiol or 1 to 2 mg of oestradiol and 1 to 5 mg of the gestagen component and optionally conventional auxiliary substances.

8. Use according to any one of claims 5 to 7, **characterised in that** a daily unit is produced by using a hormone combination consisting of in each case 15 µg, 20 µg or 30 µg of ethinyl oestradiol or in each case 1 or 2 mg of oestradiol and in each case 1, 2, 3, 4 or 5 mg of the gestagen component and optionally conventional auxiliary substances.

9. Use according to any one of claims 5 to 8, **characterised in that** a daily unit is produced by using a hormone combination of in each case 20 µg of ethinyl oestradiol and 2, 3, 4 or 5 mg of the gestagen component.

10. Use according to any one of claims 5 to 9, **characterised in that** the medicament is provided in the form of at least 21 daily units containing the hormone combination as active ingredient combination for uninterrupted administration optionally followed by a 7 to 3 day interval in taking or by 7 to 3 hormone-free daily units for uninterrupted administration.

11. Use according to claim 10, **characterised in that** the medicament is provided in the form of daily units containing one of the hormone combinations as active ingredient combination for uninterrupted administration over several years, preferably for up to 2 years, particularly preferably for up to 1 year, followed by 7 to 3 hormone-free daily units for uninterrupted administration or followed by an interval in taking of 3 to 7 days.

12. Use according to claim 11, **characterised in that** the medicament is provided in the form of 77 to 193 or of 42 to 52 or of 21 to 25 daily units containing one of the hormone combinations as active ingredient combination for uninterrupted administration followed by 7 to 3 hormone-free daily units for uninterrupted administration or followed by an interval in taking of 3 to 7 days.

13. Use according to any one of claims 10 to 12, **characterised in that**, in each daily unit containing one of the hormone combinations as active ingredient combination, the medicament comprises quantitatively the same combination of oestrogen component and gestagen component.

14. Use according to any one of claims 1 to 13 for inhibiting the proliferation of human melanocytes.

15. Use according to any one of claims 1 to 13 for reducing the melanin content in human melanocytes.

## Revendications

1. Utilisation d'au moins un progestatif choisi parmi l'acétate de chlormadinone, la 3α-hydroxy-6-chloro-17α-acétoxy-4,6-prégnadién-20-one (acétate de 3α-hydroxy-chlormadinone) et la 3β-hydroxy-6-chloro-17α-acétoxy-4,6-prégnadién-20-one (acétate de 3β-hydroxy-chlormadinone) en tant que composant progestatif et éventuellement d'au moins un oestrogène choisi parmi l'éthinylestradiol, l'estrone, l'estriol et l'estradiol en tant que composant oestrogène, pour la fabrication d'un médicament destiné au traitement et/ou à la prophylaxie du mélasme chez la femme.

2. Utilisation selon la revendication 1, **caractérisée en ce que** le médicament est présenté sous forme d'unités journalières.

3. Utilisation selon la revendication 1 ou 2, **caractérisée en ce que** pour la fabrication d'une unité journalière on utilise comme seule substance active hormonale un composant progestatif choisi parmi l'acétate de chlormadinone, l'acétate de 3α-hydroxy-chlormadinone et l'acétate de 3β-hydroxy-chlormadinone en une quantité de 1 à 10 mg, de préférence 2 à 5 mg, de façon particulièrement préférée 1, 2, 3, 4 ou 5 mg et éventuellement des adjuvants usuels.

4. Utilisation selon la revendication 3, **caractérisée en ce que** le médicament est présenté sous forme de 28, 56, 84, 112, 140, 168, 196, 224, 252, 280, 308, 336 ou 364 unités journalières pour l'administration orale journalière non interrompue.

5. Utilisation selon la revendication 1, **caractérisée en ce qu'**on utilise comme association de substances actives une association d'hormones constituée d'au moins un progestatif choisi parmi l'acétate de chlormadinone, la 3α-hydroxy-6-chloro-17α-acétoxy-4,6-prégnadién-20-one (acétate de 3α-hydroxy-chlormadinone) et la 3β-hydroxy-6-chloro-17α-acétoxy-4,6-prégnadién-20-one (acétate de 3β-hydroxy-chlormadinone) en tant que composant progestatif et d'au moins un oestrogène choisi parmi l'éthinylestradiol, l'estrone, l'estriol et l'estradiol en tant que composant oestrogène.

6. Utilisation selon la revendication 5, **caractérisée en ce que** pour la fabrication d'une unité journalière on utilise une association d'hormones constituée de 5 à 50 µg d'éthinylestradiol, 0,5 à 3 mg d'estradiol, 0,5 à 50 mg d'estrone ou 0,5 à 50 mg d'estriol et 1 à 5 mg du composant progestatif et éventuellement des adjuvants usuels.

7. Utilisation selon la revendication 5 ou 6, **caractérisée en ce que** pour la fabrication d'une unité journalière on utilise une association d'hormones constituée de 5 à 30 µg d'éthinylestradiol ou 1 à 2 mg d'estradiol et 1 à 5 mg du composant progestatif et éventuellement des adjuvants usuels.

8. Utilisation selon l'une quelconque des revendications 5 à 7, **caractérisée en ce que** pour la fabrication d'une unité journalière on utilise une association d'hormones constituée de respectivement 15 µg, 20 µg ou 30 µg d'éthinylestradiol ou respectivement 1 ou 2 mg d'estradiol et respectivement 1, 2, 3, 4 ou 5 mg du composant progestatif et éventuellement des adjuvants usuels.

9. Utilisation selon l'une quelconque des revendications 5 à 8, **caractérisée en ce que** pour la fabrication d'une unité journalière on utilise une association d'hormones constituée de respectivement 20 µg d'éthinylestradiol et 2, 3, 4 ou 5 mg du composant progestatif.

10. Utilisation selon l'une quelconque des revendications 5 à 9, **caractérisée en ce que** le médicament est présenté sous forme d'au moins 21 unités journalières contenant l'une des associations d'hormones en tant qu'association de substances actives pour l'administration ininterrompue, éventuellement suivies d'un arrêt de prise pendant 7 à 3 jours ou de 7 à 3 unités journalières sans hormones, pour l'administration ininterrompue.

11. Utilisation selon la revendication 10, **caractérisée en ce que** le médicament est présenté sous forme d'unités journalières contenant l'une des associations d'hormones en tant qu'association de substances actives pour une administration ininterrompue pendant plusieurs années, de préférence jusqu'à 2 ans, de façon particulièrement préférée jusqu'à 1 an, suivies de 7 à 3 unités journalières sans hormones, pour l'administration ininterrompue, ou suivies d'un arrêt de prise pendant 3 à 7 jours.

12. Utilisation selon la revendication 11, **caractérisée en ce que** le médicament est présenté sous forme de 77 à 193 ou de 42 à 52 ou de 21 à 25 unités journalières contenant l'une des associations d'hormones en tant qu'association de substances actives pour l'administration ininterrompue, suivies de 7 à 3 unités journalières sans hormones, pour l'administration ininterrompue, ou suivies d'un arrêt de prise pendant 3 à 7 jours.

13. Utilisation selon l'une quelconque des revendications 10 à 12, **caractérisée en ce que** le médicament comporte dans chaque unité journalière contenant l'une des associations d'hormones en tant qu'association de substances actives quantitativement la même association de composant oestrogène et de composant progestatif.

14. Utilisation selon l'une quelconque des revendications 1 à 13, pour l'inhibition de la prolifération de mélanocytes humains.

15. Utilisation selon l'une quelconque des revendications 1 à 13, pour la réduction de la teneur en mélanine de mélanocytes humains.
